# EUROPÄISCHE PATENTSCHRIFT

(11) **EP 2 736 485 B1**
(45) Veröffentlichungstag und Bekanntmachung des Hinweises auf die Patenterteilung: **28.08.2019**
(21) Anmeldenummer: 12741334.2
(22) Anmeldetag: 30.07.2012
(51) Int. Cl.: A61K 9/107, A61K 38/13, A61K 47/10, A61K 47/14, A61K 9/00

(54) **WÄSSRIGE LÖSUNGEN VON LIPOPHILEN SUBSTANZEN, INSBESONDERE ARZNEISTOFFLÖSUNGEN**
AQUEOUS SOLUTIONS OF LIPOPHILIC SUBSTANCES, IN PARTICULAR MEDICINAL SOLUTIONS
SOLUTIONS AQUEUSES DE SUBSTANCES LIPOPHILES, EN PARTICULIER DE SOLUTIONS MÉDICAMENTEUSES

(30) Priorität: 29.07.2011 DE 102011108948
(43) Veröffentlichungstag der Anmeldung: 04.06.2014
(73) Patentinhaber: Universität Regensburg, 93053 Regensburg (DE)
(72) Erfinder: LUSCHMANN, Christoph, 92287 Schmidmühlen (DE); GÖPFERICH, Achim, 93161 Sinzing (DE)
(74) Vertreter: Ahrens, Gabriele
(86) Internationale Anmeldenummer: PCT/EP2012/064895
(87) Internationale Veröffentlichungsnummer: WO 2013/017571

(56) Entgegenhaltungen:
- EP-A1- 1 712 220
- WO-A1-99/32089
- WO-A1-2007/065588
- US-A1- 2002 045 601
- US-A1- 2009 286 718
- LAZZARI P ET AL: "Antinociceptive activity of @D^9-tetrahydrocannabinol non-ionic microemulsions", INTERNATIONAL JOURNAL OF PHARMACEUTICS, ELSEVIER, AMSTERDAM, NL, vol. 393, no. 1-2, 30 June 2010 (2010-06-30), pages 239-244, XP027065393, ISSN: 0378-5173, DOI: 10.1016/J.IJPHARM.2010.04.010 [retrieved on 2010-04-23]

## Beschreibung

Gegenstand der vorliegenden Erfindung sind flüssige, wässrige Formulierungen zur topischen Verwendung am Auge für lipophile, schwer wasserlösliche pharmazeutische Wirkstoffe gemäß Anspruch 1, die am menschlichen oder tierischen Körper angewendet werden können. Die flüssige, wässrige Formulierung eignet sich insbesondere zur Herstellung von ophthalmischen Zusammensetzungen.

Viele potente Arzneistoffe, welche sich zur effizienten Therapie verschiedenster Erkrankungen eignen, sind aufgrund ihrer sehr schlechten Löslichkeit in Wasser oft nicht therapeutisch einsetzbar. Diese Eigenschaft der geringen Löslichkeit lässt sich nach dem europäischen Arzneibuch definieren über die Massenanteile an Lösungsmittel, die für die Herstellung einer Lösung von einem Massenteil Substanz benötigt wird (Europäisches Arzneibuch 5.0/1.04.00.00). In diesem Zusammenhang unterscheidet man:
- schwer löslich
   *pro Teil Stoff werden 100 bis 1000 Teile Lösungsmittel benötigt*
- sehr schwer löslich
   *pro Teil Stoff werden 1000 bis 10000 Teile Lösungsmittel benötigt*
- praktisch unlöslich
   *pro Teil Stoff werden über 10000 Teile Lösungsmittel benötigt.*

Unter einer Lösung wird eine homogene Verteilungen von zwei oder mehreren unterschiedlichen Stoffen ineinander verstanden. Dabei unterscheidet man:
- echte Lösungen
- kolloidale Lösungen.

In echten Lösungen ist die gelöste Substanz molekulardispers im Lösungsmittel verteilt, d.h. sie liegt in diskreten Einzelmolekülen vor. Bei kolloidalen Lösungen befindet sich die Substanz in Beladungen bis zu 100% in Partikeln, wie zum Beispiel Mizellen, von kolloidaler Größe, d.h. zwischen 1 und 500 nm.

Von einer Lösung, die ein Einphasensystem darstellt, sind Emulsionen zu unterscheiden, die mehrphasige Systeme von zwei miteinander nicht mischbaren Flüssigkeiten sind.

Aus einer schlechten Löslichkeit des Arzneistoffs im Wässrigen resultieren diverse Probleme:
- unzureichende Wirkstoff-Konzentration am Wirkort
- Notwendigkeit der Verwendung von Lösungsmitteln mit schlechter Verträglichkeit
- Entwicklung aufwändiger Rezepturen
- erhöhte Produktionskosten.

Es sind zahlreiche Strategien zur Verbesserung der Löslichkeit in Wasser und zur Verabreichung schwerlöslicher lipophiler pharmazeutischer Wirkstoffe bekannt. So gibt es zahlreiche Vorschläge zur Herstellung stabiler Zubereitungen in Form von Lösungen oder mizellaren Systemen, mittels geeigneter Lösungsmittel, wie z.B. flüssige Polyethylenglycole, oder Lösungsvermittler, d.h. Tenside, die den Arzneistoff kolloidal komplexieren oder verkapseln, und so den Wirkstoff in Lösung halten. Beispiele hierfür finden sich u.a. in DE 102008059201 A1, US 5,474,979, EP 0945136 B9, US 6,267,985, US 6,309,663 und US 2010/0305046 A1.

DE102008059201 A1 beschreibt Zubereitungen mit flüssigen Polyethylenglycolen als Lösungsmittel, deren Wirkstoff, bevorzugt Cyclosporin A, bei Kontakt mit wässrigen Flüssigkeiten, bevorzugt der Tränenflüssigkeit, in Nanostrukturen ausfällt.

US 5,474,979 beschreibt eine Öl in Wasser Emulsion für Cyclosporin A mit 0,05% Wirkstoffgehalt. Als lipophile Phase werden Fettsäureglyceride, wie beispielsweise Rizinusöl eingesetzt. Als Lösungsvermittler dienen Tenside, wie Polysorbat 80, ein Polyoxyethylensorbitanfettsäureester. Die Öl in Wasser Emulsion wird bevorzugt am Auge appliziert und soll über 9 Monate stabil sein.

Eine Nanoemulsion für Cyclosporin A wird in EP 0945136 B9 beschrieben. Unter Verwendung von Triglyeriden als lipophiler Phase und mindestens einem Phospholipid, bevorzugt Lecithin, wird hier eine stabile Formulierung erzielt.

Ähnlich verhält es sich für US 6,267,985. Deren Gegenstand betrifft eine verbesserte Löslichkeit bzw. Dispergierbarkeit von Triglyceriden als liphophiler Phase in wässrigen Flüssigkeiten und eine dadurch gesteigerte Applizierbarkeit von Arzneistoffen. Diese erhöhte Löslichkeit, Verteilung und Stabilität wird durch eine Kombination von mindestens zwei Tensiden gewährleistet, wobei mindestens ein Tensid hydrophil ist.

In US 6,309,663 werden ebenfalls mindestens zwei Lösungsvermittler eingesetzt, wovon einer hydrophil und der andere hydrophob ist. Hier handelt es sich um ein triglyceridfreies System, allerdings für hydrophile Wirkstoffe. Die Kombination der Tenside soll eine verbesserte Resorption, d.h. verbesserte Wirkstoffaufnahme, in Zellen und Gewebe zur Folge haben.

Eine stabile Emulsion zum Einsatz am Auge, welche Cyclosporin enthält, beschreibt auch US 2010/0305046 A1. Dabei wird die O/W-Emulsion mit einer Partikelgröße der inneren Phase von kleiner 1µm einstellt. Zur Verbesserung der Löslichkeit wird ein System beschrieben, das aus mindestens einem Öl und einer Kombination aus hydrophilem Emulgator aus der Gruppe der Polyethylenoxidfettalkoholether (Brij) und einem pflanzlich gewonnenen, hydrophoben Emulgator mit ungesättigten Seitenketten. Das eingesetzte Cyclosporin ist bevorzugt Cyclosporin A.

Alle aufgeführten Beispiele beschreiben Systeme, welche entweder auf ölige Phasen zurückgreifen, oder bei Verzicht auf diese, hydrophile und damit in Wasser gut lösliche Wirkstoffe applizieren bzw. mehrere Lösungsvermittler benötigen, um die Löslichkeit des Arzneistoffs und die Stabilität der Formulierung zu verbessern.

Eine liposomale Formulierung wird in WO 2007/065588 dargestellt. In wässrigem Millieu werden hierzu Gemische von Lösungsvermittlern aus der Gruppe der Phospholipide und nicht-ionischen Emulgatoren wie Polyoxyethylenalkylether und Polyoxyethylensorbitanfettsäureester, gewählt, um eine stabile Verteilung des Arzneistoffs zu erhalten. Der Arzneistoff ist hierbei erneut Cyclosporin A und bevorzugt liposomal eingeschlossen. Das Produkt ist ein flüssiges System.

EP 1 712 220 A1 beschreibt eine pharmazeutische Aerosolzusammensetzung mit einem schwer wasserlöslichen Wirkstoff, wobei zur Lösung des Wirkstoffes zwei Tenside eingesetzt werden, ein nichtionisches Tensid und ein Phospholipid.

US 2002/0045601 A1 beschreibt ophthalmische Zusammensetzungen mit Cyclosporin, einem schwer wasserlöslichen Wirkstoff. Für die Herstellung ist es erforderlich den Wirkstoff zunächst in Ethanol beziehungsweise Propylenglycol als Cosolvent vorzulösen und dann die erhaltene Lösung Wasser zuzusetzen.

US 2009/0286718 A1 betrifft ebenfalls ophthalmische Zusammensetzungen mit Cyclosporin als schwer wasserlöslichem Wirkstoff, wobei auch hier der Wirkstoff zunächst in Ethanol als Cosolvent vorgelöst und dann die erhaltene Lösung Wasser zugesetzt wird.

WO 99/32089 A1 betrifft pharmazeutische Zusammensetzungen mit einem schwer wasserlöslichem lipophilen Glucocorticoid für die Behandlung von allergischen und entzündlichen Erkrankungen des Atemtrakts sowie zur Behandlung des Darmtrakts.

Die wässrige Basislösung wird durch Einrühren von Wasser in eine Mischung aus einem einzigen Tensid und dem Glucocorticoid erhalten.

LAZZARI P ET AL, Antinociceptive activity of Δ9-tetrahydrocannabinol non-ionic microemulsions, in Int. J. Pharm. 393 (2010) 238-243, beschreibt eine wässrige pharmazeutische Zusammensetzung mit Δ⁹-tetrahydrocannabinol, ein schwer wasserlöslicher Wirkstoff, für die parenterale (intraperitoneale und intergastrale) Verabreichung, wobei die wässrige Basislösung durch Vermischen von Solutol® HS15 als Tensid, Wirkstoff und Wasser erhalten wird.

Wird ein lipophiler Wirkstoff in lipophilen Lösungsmitteln gelöst, ist es sehr viel schwieriger diesen am Wirkort in geeigneten Konzentrationen zur Resorption bereitzustellen, da die Bindung im Lösungsmittel zu stark ist. Dies ist sowohl bei Emulsionen, als auch rein öligen Systemen der Fall. Damit kann es zu Schwankungen der Bioverfügbarkeit des Arzneistoffs und mangelnder Genauigkeit der Dosierung, bzw. Fehldosierungen kommen. Bevorzugt gelangen Stoffe an ihre Zielstrukturen, wie beispielsweise Rezeptoren an der Zelloberfläche, wenn diese in wässrigem Millieu gelöst sind. Die Zusammensetzung der Zubereitung spielt hierfür also eine herausragende Rolle.

Die Zusammensetzung der Formulierung spielt neben der Resorption gerade auch hinsichtlich der Komplexität der Produktion, sowie der damit verbundenen Kosten eine sehr gewichtige Rolle. Wird auf ölige Bestandteile verzichtet, was im Rahmen der Herstellung und Anwendung vorteilhaft ist, bedarf es in der Regel zweier oder mehrerer Lösungsvermittler, um ausreichende Konzentrationen und Stabilität der Wirkstoffe im Wässrigen zu gewährleisten.

Bekannt ist ein wässriges System, das mit nur einem Lösungsvermittler auskommt. Bei dem Lösungsvermittler handelt es sich um eine speziell entwickelte Gruppe von Tensiden, MPEG-hexPLA-Blockcopolymere.

Bislang ist aber in beiden Fällen, egal ob ein oder mehrere Lösungsvermittler eingesetzt werden, immer eine äußerst aufwendige Herstellung damit verbunden. D.h. es werden beispielsweise separate Lösungen mit Lösungsvermittler und Arzneistoff in unterschiedlichen Lösungsmitteln, wie z.B. Wasser, Ethanol, usw. angesetzt, danach vermengt, um abschließend über Evaporation das organische Lösungsmittel wieder zu entfernen.

Ein aus nur wenigen Komponenten bestehendes System, welches ohne große Umwege, beispielsweise nur durch Vermengen und Rühren hergestellt werden kann, würde die Produktion, sowie deren Reproduzierbarkeit und Qualität erheblich verbessern und deren Kosten minimieren.

Grundvoraussetzung, um die oben genannten Probleme zu umgehen ist, dass die Substanzen in einer geeigneten Arzneiform so an den Applikationsort gebracht werden können, dass sie dort möglichst homogen, d.h. gleichmäßig und fein verteilt, vorliegen, damit sie effektiv resorbiert werden können. Dadurch wird gewährleistet, dass eine im Rahmen der Löslichkeit möglichst hohe Konzentration rasch und dauerhaft verfügbar wird.

Um dieses Ziel zu erreichen sind dem Fachmann zahlreiche Verfahren bekannt. Die einfachste, jedoch sehr ungünstige Strategie ist, wie bereits erwähnt, das Lösen des Wirkstoffs in Ölen, wie z.B. flüssigen Triglyceriden, wie man sie in zahlreichen Pflanzenölen findet, oder wie sie als Neutralöl unter dem Handelsnamen Miglyol bekannt sind. Zwar können solche Lösungen am Auge und auch parenteral, d.h. z. B. in Form von Injektionen, angewendet werden, doch haben sie die oben aufgeführten Nachteile. Darüber hinaus können sich Probleme mit der Verträglichkeit mit dem Gewebe am Applikationsort ergeben. Die Applikation öliger Lösungen ist häufig von Entzündungsreaktionen begleitet. Im Rahmen der Anwendung am Auge kommt es teilweise zu einer Beeinträchtigung des Sehvermögens durch Schlierenbildung, die darauf zurückzuführen ist, dass sich das Öl mit der Tränenflüssigkeit nicht mischt. Darüber hinaus ist die Verweilzeit am Auge unzureichend.

Um die Verfügbarkeit des Arzneistoffs zu verbessern, bietet es sich speziell für Anwendungen am Auge an, Salben im Sinne des Arzneibuchs einzusetzen (Europäisches Arzneibuch 5.0, Halbfeste Zubereitungen zur kutanen Anwendung 5.0/0123 und Halbfeste Zubereitungen zur Anwendung am Auge 5.0/1163).

Zwar ist deren Verweilzeit am Auge erhöht, jedoch erfordert deren sterile Herstellung einen aseptisch angelegten Produktionsvorgang, was die Kosten gegenüber einer Lösung, die sterilfiltriert werden kann, stark erhöht. Zudem gehen diese Zubereitungen mit einer sehr geringen Compliance einher, da der Patient Fremdkörpergefühl und eine substanzielle temporäre Sichtbehinderung durch Schlierenbildung erdulden muss. Die parenterale Applikation solcher Systeme scheitert darüber hinaus häufig an der geringen Fluidität der Systeme, die eine Passage durch eine Kanüle verhindert oder erschwert.

Suspensionen bzw. Nanosuspensionen lipophiler Arzneistoffe, in denen die Arzneistoffe als Feststoffteilchen verteilt vorliegen, bieten die Möglichkeit, den oben skizzierten Anforderungen an die Applikation gerecht zu werden, indem sie für eine "feine" und homogene Verteilung des Arzneistoffs am Applikationsort sorgen. Dem steht jedoch eine extrem aufwändige Herstellung gegenüber. Insbesondere bei Suspensionen kommt die geringe Lagerstabilität aufgrund von Agglomeration oder Sedimentation der Arzneistoffteilchen hinzu, die mit großem Aufwand so lange als möglich unterdrückt werden müssen.

Kolloidale Systeme, wie Liposomen, bieten grundsätzlich einen sehr vielversprechenden Ansatz. Jedoch sind die bislang bekannten Formulierungen komplex, aufwändig in der Herstellung und zeigen oft nur geringe Wirkstoffbeladung oder Stabilität.

Die bislang aussichtsreichste Möglichkeit lipophile Arzneistoffe zu formulieren und anzuwenden sind daher, wie vorstehend ausgeführt mit Cyclosporin A als Wirkstoff, Emulsionen. Mit Restasis® ist in den USA bereits ein Augentropfenpräparat auf dem Markt, welches effektive Wirkstoffspiegel bei mehrmaliger täglicher Applikation liefert. Doch auch hier stellen hohe Produktions- und Entwicklungskosten, sowie unzureichende Haltbarkeit und Stabilität des Systems bei Anwesenheit von Wasser ein Problem dar. Ein weiterer Nachteil ist, dass der Patient das Präparat, wie zum Beispiel im Fall von Restasis®, selbst für die Applikation vorbereiten und die Emulsion redispergieren muss, so dass der Therapieerfolg von der korrekten Vorbereitung und Anwendung des Patienten abhängt. Bedingt durch das System "Emulsion" liegt das Cyclosporin A auch hier in einer lipophilen Phase gelöst vor, was, wie zuvor beschrieben, für die Resorption wiederum von Nachteil ist.

Wünschenswert ist daher eine Formulierung, welche lipophile Substanzen in wässrigen Flüssigkeiten löst, dazu aber nicht auf ölige Lösungsmittel bzw. Cosolventien zurückgreift. Der Patient selbst soll vor Applikation keinerlei Einfluss, wie beispielsweise Aufschütteln, mehr auf das Produkt nehmen müssen und es direkt anwenden können. Bei der Applikation sollen keinerlei unerwünschten Nebenwirkungen, wie Brennen oder Schlierenbildung auftreten und der Wirkstoff soll gleichmäßig verteilt und resorbiert werden. Weiter soll das System einfach und kostengünstig zu produzieren und mittels Filtration sterilisierbar sein. Ein solches System würde in der Therapie am Auge einen hohen Anwendungskomfort gewährleisten, da es am Auge durch Eintropfen ohne Schlierenbildung oder Brennen einfach und für den Organismus gut verträglich applizierbar wäre.

Es war daher Aufgabe der vorliegenden Erfindung ein verbessertes System zur Verfügung zu stellen, das die vorstehend genannten Anforderungen erfüllt, mit dem die Bioverfügbarkeit - also die Geschwindigkeit und das Ausmaß, mit denen der Wirkstoff aus der Zubereitung absorbiert wird oder am Wirkort vorliegt - von lipophilen, schwer bis praktisch in Wasser unlöslichen Wirkstoffen am oder im menschlichen oder tierischen Körper erhöht werden kann, und das auf einfache, kostensparende Weise, z. B. mittels Sterilfiltration, erhältlich ist.

Erfindungsgemäß wird diese Aufgabe gelöst durch eine wässrige Lösung zur topischen Verwendung am Auge bestehend aus mindestens einem lipophilen pharmazeutischen Wirkstoff, ausgewählt unter Cyclosporin A, Budesonid, Beclometason, Triamcinolonacetonid, Dexamethason oder Fluticasondipropionat, einem Tensid ausgewählt aus der Gruppe der Polyoxyethylenfettalkoholether und deren Derivaten, Polyoxyethylenfettsäureester und deren Derivaten sowie kationischen Tensiden, und Wasser, wobei die wässrige Lösung durch Verrühren des mindestens einen pharmazeutischen Wirkstoffes, des Tensids und Wasser zueinander erhalten wird.

Erfindungsgemäß wurde gefunden, dass ein Tensid ausgewählt aus der Gruppe der Polyoxyethylenfettalkoholether und deren Derivaten, Polyoxyethylenfettsäureester und deren Derivaten und kationischen Tenside in der Lage ist, einen schwerlöslichen lipophilen Wirkstoff, in einem wässrigen Milieu zu lösen. Besonders vorteilhaft ist, dass es ausreichend ist, einen einzigen Typ eines Tensids einzusetzen. Anders als im Stand der Technik diskutiert, ist es nicht mehr erforderlich, mehrere Lösungsmittel und/oder eine Kombination von zwei oder mehreren verschiedenen Typen von Tensiden als Lösungsvermittler zu verwenden.

Ein weiterer Vorteil ist, dass eine hohe Wirkstoffbeladung erhalten werden kann, das heißt, das erfindungsgemäß eingesetzte Tensid ist in der Lage, einen hohen Anteil des zugesetzten lipophilen Wirkstoffs kolloidal zu lösen und zu solubilisieren. Ein weiterer Vorteil ist, dass die erfindungsgemäße wässrige Lösung auf einfache Art und Weise durch Einrühren in Wasser und anschließendem Sterilfiltrieren hergestellt werden kann.

Die erfindungsgemäße wässrige Lösung enthält feinverteilte, mit dem lipophilen Wirkstoff beladene Mizellen. Die erfindungsgemäße Lösung ist eine kolloidale Lösung.

Die erfindungsgemäße Lösung ist ganz besonders geeignet ist für die Applikation lipophiler Arzneistoffe am Auge.

Mit der vorliegenden Erfindung ist es somit möglich, lipophile Wirkstoffe als Lösungen zu applizieren, ohne auf "ölige" Lösungsmittel oder andere lipophile Lösungsmittel wie Triglyceride oder Cholesterol zurückzugreifen. Diese öligen oder lipophilen Lösungsmittel würden im Gegensatz zur erfindungsgemäßen Lösung mit Wasser eine Emulsion, d. h. ein zweiphasiges System, ausbilden. Insbesondere ist es mit dem erfindungsgemäßen System möglich, auch ohne lipophile Lösungsmittel, eine durchweg einfache Herstellung zu gewährleisten. Daher eignet sich das erfindungsgemäße System aufgrund seiner Einfachheit im Herstellungsprozess auch für industrielle Größenordnungen.

Erfindungsgemäß wird als Lösungsmittel Wasser eingesetzt. Die für die Erfindung eingesetzten Tenside sind in der Lage lipophile Substanzen zu lösen, so dass eine kolloidale Lösung entsteht. Es werden Tenside eingesetzt, die physiologisch verträglich und nicht toxisch sind. Besonders bevorzugt sind Tenside, die mit Wasser transparente, farblose und wenig viskose Lösungen ergeben.

Insbesondere können die erfindungsgemäß eingesetzten Tenside eine wässrige kolloidale Lösung bilden, in der sich die mindestens eine lipophile Substanz in Beladungen bis zu 100 % in den Mizellen von kolloidaler Größe, das heißt zwischen 1 und 500 nm, vorliegen. Eine Beladung von bis zu 100 % liegt vor, wenn die gesamte zugesetzte lipophile Substanz in Form von Mizellen gelöst und solubilisiert ist.

Erfindungsgemäß ist es ausreichend die Tenside in einer Konzentration einzusetzen, die kleiner als 20 % (m/v), ist. Vorzugsweise ist die Konzentration kleiner 10 %, bevorzugt kleiner 5 % und besonders bevorzugt kleiner 2 % (m/v). Jedoch richtet sich die Konzentration an Tensid nach dem zu lösenden lipophilen Wirkstoff, insbesondere dessen Löslichkeit und Menge.

Erfindungsgemäß werden als Tenside Polyoxyethylenfettalkoholether und deren Derivate, Polyoxyethylenfettsäureester und deren Derivate und kationische Tenside eingesetzt.

Beispiele für geeignete Polyoxyethylenfettalkokolether und Polyoxyethylenfettsäureester sind diejenigen auf Basis von Fettalkoholen beziehungsweise von Fettsäuren mit C11-C18 und 1 - 25 Ethylenglycoleinheiten. Es können sowohl die Mono- als auch die Diester als Tenside für die vorliegende Erfindung eingesetzt werden.

Beispiele für geeignete Fettalkohole sind Laurylakohol mit C12, Myristylalkohol mit C14, Cetylalkohol mit C16 und Stearylalkohol mit C18.

Beispiele für geeignete Fettsäuren sind Laurinsäure mit C12, Myristinsäure mit C14, Palmitinsäure mit C16 und Stearinsäure mit C18.

Beispiele für Polyoxyethylenfettalkoholether wie sie für die vorliegende Erfindung geeignet sind, sind Polyoxyethylenlaurylether mit 4, 9 beziehungsweise 23 Ethylenglycolmonomeren, Polyoxyethylencetylether mit 2, 10, 20 Ethylenglycolmonomeren, Polyoxyethylenether des Cetylstearylalkohols (16/18 C-Atome), die auch unter der Bezeichnung Macrogolcetylstearylether bekannt sind, mit 6, 20 beziehungsweise 25 Ethylenglycolmonomeren (INCI: Ceteareth-6, Ceteareth-20 und Ceteareth-25), Polyoxyethylenstearylether mit 2, 10 beziehungsweise 20 Ethylenglycolmonomeren, und Polyoxyethylenoleylether mit 2, 10 beziehungsweise 20 Ethylenglycolmonomeren.

Beispiele für Polyoxyethylenfettsäureester sind 2-Hydroxyethylstearat, Polyoxyethylenester der Stearinsäure mit 8 Ethylenglycolmonomeren, Polyoxyethylenester der 12-Hydroxystearinsäure mit 15 Ethylenglycolmonomeren (Solutol® HS15 hergestellt von der Firma BASF; chemische Bezeichnung: Macrogol-15-Hydroxstearat).

Beispiele für kationische Tenside sind Polyethylenglycol-Polylactid-Blockcopolymere (PEG-PLA-Blockcopolymere), die vorzugsweise an den hydrophilen Enden mit positiv geladenen Gruppen funktionalisiert sind. Geeignete PEG-PLA-Blockcopolymere sind zum Beispiel in DE 199 30 729 A1 beschrieben, auf die hier ausdrücklich verwiesen wird.

Ein Beispiel für eine positiv geladene Gruppe wie sie für die vorstehend genannten PEG-PLA-Blockcopolymere eingesetzt werden können, sind positiv geladene Amingruppen.

Geeignete kationische Tenside sind auch quartäre Ammoniumsalze wie zum Beispiel Alkyltrimethylammoniumsalze mit einer linearen gesättigten oder ungesättigten C6 bis C16 Alkylkette.

Erfindungsgemäß besonders bevorzugte Tenside sind diejenigen mit der chemischen Bezeichnung Macrogol-15-Hydroxystearat (Handelsbezeichnung Solutol® HS15), Steareth 20 (Handelsbezeichnung Sympatens AS), Ceteareth 20 (Handelsbezeichnung Sympatens ACS) sowie Ceteareth 25 (Handelsbezeichnung Cremophor A25).

Die vorliegende Erfindung bezieht sich auf lipophile pharmazeutische Arzneistoffe gemäß Anspruch 1.

Beispiele hierfür sind Substanzen wie Cyclosporin A, Budesonid, Beclomethason, Triamcinolonacetonid, Dexamethason, Fluticasondipropionat. Ganz besonders bevorzugt sind Cyclosporin A und Budesonid.

Erfindungsgemäß können zum Beispiel Cyclosporin A in einer Konzentration bis zu 0,05 %, insbesondere bis zu 1,0 % und besonders bevorzugt bis zu 3,0 % (m/v) und Budesonid in einer Konzentration bis zu 0,025 %, insbesondere bis zu 0,05 % und insbesonders bevorzugt bis zu 0,1 % (m/v), in Lösung gebracht werden.

Das einfache grundlegende System besteht aus einem erfindungsgemäß geeigneten Tensid, einem lipophilen Wirkstoff und Wasser. Durch Lösen der Bestandteile mittels Rühren entsteht ein vorzugsweise klares, kolloidales System, in welchem die lipophile Substanz in Mizellen verkapselt gelöst ist. Insbesondere lassen sich mit den erfindungsgemäß eingesetzten Tensiden kolloidale Systeme mit Mizellen einer Größe von kleiner 30 nm erhalten. Transparente und flüssige Systeme lassen sich zum Beispiel sowohl für Budesonid, wie für Cyclosporin A auch analog zu Macrogol-15-hydroxystearat Solutol® HS15 mit Steareth 20 (Sympatens AS) und Ceteareth (Sympatens ACS) formulieren. Hier ist es bereits mit 5 % (m/v) Tensid möglich mindestens 1 % (m/v) Cyclosporin A zu lösen. Dies entspricht weniger als einem Viertel der Menge, die an Solutol benötigt wird.

Nachstehend werden Beispiele für unterschiedliche Ausführungsformen der vorliegenden Erfindung aufgeführt, anhand derer gezeigt wird, wie der Einsatz der erfindungsgemäß eingesetzten Tenside in unterschiedlichen Konzentrationen die Lösungseigenschaften von Wirkstoffen verbessern können.

Soweit nicht anders angegeben, beziehen sich die Prozentangaben auf (m/v). Als Lösungsvermittler wurden folgende Tenside eingesetzt:
- Sympatens AS-INCI: Steareth 20 (ein Polyoxyethylenfettsäureester der Stearinsäure mit 20 Etylenglycol-Einheiten);
- Sympatens ACS-INCI: Ceteareth 20, (ein Polyoxyethylenfettsäurealkoholether des Cetylstearylalkohols (16/18 C-Atome) mit 20 Ethylenglycoleinheiten); sowie
- Solutol HS15, chemische Bezeichnung: Macrogol-15-Hydroxystearat (ein Polyoxyethylenfettsäureester der Stearin-Säure, wobei Mono- und Diester mit 8-20 Ethylenglycoleinheiten (im Durchschnitt 15) vorliegen).

### Beispiel 1: Lösung aus Cyclosporin A, Solutol® HS15 und Wasser

| | | | | | | |
|---|---|---|---|---|---|---|
| Cyclosporin A | *mg* | 15,2 | 1,26 | 0,86 | 0,46 | 0,12 |
| Solutol® HS15 | *mg* | 300,00 | 200,00 | 100,00 | 50,00 | 10,0 |
| Aqua bidest. | *ml* | ad 1,00 | ad 1,00 | ad 1,00 | ad 1,00 | ad 1,00 |

Wirkstoff und Tensid wurden eingewogen und um 1ml Aqua bidest. ergänzt. Dieser Ansatz wurde über Nacht durch Rühren bei Raumtemperatur (21°C) gelöst. Diese Formulierung ergab ein transparentes, leicht viskoses kolloidales System mit einer durchschnittlichen Mizellgröße von 14 nm. Die Messungen wurden mittels Photonenkorellationsspektrometrie durchgeführt.

### Beispiel 2: Lösung aus Budesonid, Solutol® HS15 und Wasser

| | | | | | |
|---|---|---|---|---|---|
| Budesonid | *mg* | 1,32 | 0,77 | 0,41 | 0,09 |
| Solutol® HS15 | *mg* | 200,00 | 100,00 | 50,00 | 10,00 |
| Aqua bidest. | *ml* | ad 1,00 | ad 1,00 | ad 1,00 | ad 1,00 |

Die Herstellung erfolgte analog wie bereits unter Beispiel 1 beschrieben. Auch diese Formulierungen ergaben transparente, leicht viskose Systeme mit einer durchschnittlichen Mizellgröße von 14 nm.

### Beispiel 3: Löslichkeit und Stabilität mittels Sympatens ACS, Sympatens AS und Solutol® HS15

Es wurden drei wässrige Zubereitungen mit (a) 5% (m/v) Sympatens AS, (b) 5 % (m/v) Sympatens ACS, (c) 15 % (m/v) Solutol und jeweils 1 % (m/v) Cyclosporin A bzw. 0,1 % Budesonid hergestellt. Diese Zubereitung wurde über Nacht bei Raumtemperatur (21°C) gerührt. Die fertigen Systeme waren transparent, nur leicht viskos und hatten vom Wirkstoff unabhängig Mizellgrößen von (a) 9 nm, (b) 9 nm und (c) 13 nm. Sie wurden bei 5°C 1 Jahr (Cyclosporin A) bzw. 9 Monate (Budesonid) gelagert und zu definierten Zeitpunkten wurde jeweils eine Probe gezogen. Die quantitative Analytik mittels Hochdruck-Flüssigkeits-Chromatographie zeigte keinerlei Änderungen im Gehalt der Proben. Ebenso wurden mittels dynamischer Lichtstreuung keine Änderungen in Mizellgröße und Größenverteilung verzeichnet. Sie waren mindestens über diesen Zeitraum stabil.

### Beispiel 4: Verträglichkeit an primären epithelialen Cornea Zellen

Es wurden drei wässrige Formulierungen von (a) 5 % Sympatens AS, (b) Sympatens ACS und (c) 10% Solutol® HS15 auf ihre Verträglichkeit an primären epithelialen Cornea Zellen getestet. Dazu wurde diese Systeme euhydrisch und isoton eingestellt. Die Formulierungen wurden in einer Durchfluss Apparatur über die Zellen geleitet und mittels Kamera die Veränderung der Zellfläche dokumentiert. Als Standard wurde eine auf dem Markt befindliche Augentropfen Formulierung mit Na-Hyaluronat verwendet. Die Zellen verhielten sich unter Einfluss der Proben ebenso, wie unter dem Einfluss der Referenz. Die Formulierungen zeigten keine Nachteile gegenüber der Referenz.

## Patentansprüche

1. Wässrige Lösung zur topischen Verwendung am Auge bestehend aus mindestens einem lipophilen pharmazeutischen Wirkstoff, ausgewählt unter Cyclosporin A, Budesonid, Beclometason, Triamcinolonacetonid, Dexamethason oder Fluticasondipropionat,
einem Tensid ausgewählt aus der Gruppe der Polyoxyethylenfettalkoholether und deren Derivaten, Polyoxyethylenfettsäureester und deren Derivaten sowie kationischen Tensiden, und Wasser,
**wobei** die wässrige Lösung durch Verrühren des mindestens einen pharmazeutischen Wirkstoffes, des Tensids und Wasser zueinander erhalten wird.

2. Wässrige Lösung nach Anspruch 1,
**wobei** der mindestens eine pharmazeutische Wirkstoff Cyclosporin A oder Budesonid ist.

3. Wässrige Lösung nach einem der Ansprüche 1 oder 2,
**wobei** die Fettsäure beziehungsweise der Fettalkohol ein C11 bis C18 - Fettalkohol beziehungsweise Fettsäure ist und die Polyoxyethylenkette 1 bis 25 Ethylenglycolmonomere aufweist.

4. Wässrige Lösung nach einem der Ansprüche 1 bis 3,
**wobei** das kationische Tensid ausgewählt ist aus der Gruppe der Polyethylenglycol-Polylactide, die mit positiv geladenen Gruppen modifiziert sind, und der quartären Ammoniumsalze.

5. Wässrige Lösung nach einem der Ansprüche 1 bis 4,
**wobei** durch Lösen der Bestandteile mittels Rühren eine kolloidale Lösung erhalten wird, in der der mindestens eine lipophile pharmazeutische Wirkstoff in Mizellen verkapselt gelöst vorliegt.

## Claims

1. Aqueous solution for topical administration at the eye, containing at least one lipophilic pharmaceutical active agent, selected from the group of cyclosporine A, budesonide, beclometasone, triamcinolone acetonide, dexamethasone and fluticasone dipropionate,
a surfactant selected from the group of polyoxyethylene fatty alcohol ethers and derivatives thereof, polyoxyethylene fatty acid esters and derivatives thereof as well as cationic surfactants, and water,
**wherein** the aqueous solution will be obtained by mixing the at least one pharmaceutical active agent, the surfactants, and water with each other.

2. Aqueous solution according to claim 1,
**wherein** the at least one pharmaceutical active agent is cyclosporine A or budesonide.

3. Aqueous solution according to claims 1 or 2,
**wherein** the fatty acid or the fatty alcohol, respectively, is a C11 to C18 fatty alcohol or fatty acid, respectively, and the polyoxyethylene chain comprises 1 to 15 ethylene glycol monomers.

4. Aqueous solution according to any of claims 1 to 3,
**wherein** the cationic surfactant is selected from the group of polyethylene glycol polylactides which are modified with positively charged groups, and quaternary ammonium salt.

5. Aqueous solution according to any of claims 1 to 4,
**wherein** the colloidal solution is obtained by the solving of ingredients via mixing which incorporates the at least one lipophilic pharmaceutical active agent solved in micelles encapsulated.

## Revendications

1. Solution aqueuse pour l'application topique oculaire, constituée d'au moins une substance active pharmaceutique lipophile, choisie parmi la cyclosporine A, le budésonide, la béclométasone, l'acétonide de triamcinolone, la dexaméthasone ou le diproprionate de fluticasone,
d'un tensioactif choisi dans le groupe des éthers d'alcools gras de polyoxyéthylène et leurs dérivés, des esters d'acides gras de polyoxyéthylène et leurs dérivés ainsi que des tensioactifs cationiques, et d'eau,
**dans** laquelle la solution aqueuse est obtenue par mélange de l'au moins une substance active pharmaceutique, du tensioactif et de l'eau les uns avec les autres.

2. Solution aqueuse selon la revendication 1,
**dans** laquelle l'au moins une substance active pharmaceutique est la cyclosporine A ou le budésonide.

3. Solution aqueuse selon l'une quelconque des revendications 1 ou 2,
**dans** laquelle l'acide gras ou l'alcool gras est un alcool gras ou acide gras en C11 à C18 et la chaîne de polyoxyéthylène présente 1 à 25 monomères d'éthylène glycol.

4. Solution aqueuse selon l'une quelconque des revendications 1 à 3,
**dans** laquelle le tensioactif cationique est choisi dans le groupe des polylactides de polyéthylène glycol qui sont modifiés par des groupes chargés positivement et des sels d'ammonium quaternaires.

5. Solution aqueuse selon l'une quelconque des revendications 1 à 4,
**dans** laquelle une solution colloïdale, dans laquelle l'au moins une substance active pharmaceutique lipophile se présente dissoute encapsulée dans des micelles, est obtenue par dissolution des composants au moyen d'une agitation.
